(19) Europäisches Patentamt
European Patent Office
Office européen des brevets

(11) Veröffentlichungsnummer: **0 176 868**
**B1**

(12) **EUROPÄISCHE PATENTSCHRIFT**

(45) Veröffentlichungstag der Patentschrift: **12.04.89**

(51) Int. Cl.⁴: **C 07 C 127/22**, A 01 N 47/34, C 07 C 87/60, C 07 C 119/048, C 07 C 117/02, C 07 C 118/04

(21) Anmeldenummer: **85111776.2**

(22) Anmeldetag: **18.09.85**

(54) **Neue insektizid wirksame Benzoylharnstoffe.**

(30) Priorität: 29.09.84 DE 3435895
14.03.85 DE 3509075
18.07.85 DE 3525625

(43) Veröffentlichungstag der Anmeldung:
**09.04.86 Patentblatt 86/15**

(45) Bekanntmachung des Hinweises auf die Patenterteilung:
**12.04.89 Patentblatt 89/15**

(84) Benannte Vertragsstaaten:
**AT BE CH DE FR GB IT LI LU NL SE**

(56) Entgegenhaltungen:
**DE-A-2 123 236**
**DE-A-3 044 055**
**DE-A-3 123 720**
**GB-A-2 106 499**
**GB-A-2 106 501**

**CHEMICAL ABSTRACTS, Band 62, no. 12, 7. Juni 1985, Columbus, Ohio, USA, W.S. GUMP et al. "Chemical structure and antimicrobial activity of bisphenols", Spalte 14 546c**
**CHEMICAL ABSTRACTS, Band 70, no. 17, 28. April 1969, Columbus, Ohio, USA, G. BECK et al. "2,6-Dihalophenyl isothiocyanates", p. 302, Zusammenfassung-Nr. 77 561m**
**JOURNAL OF AGRICULTURAL AND FOOD CHEMISTRY, Band 21, Nr. 3, 1973, American Chemical Society, Washington, D.C., K. WELLINGÀ et al. "Synthesis and laboratory evaluation of 1-(2,6-disubstituted bezoyl)-3-phenylureas, a new class of insecticides. I. 1-(2,6-dichlorobenzoyl)-3-phenylureas", pp. 348-354**
**CHEMICAL ABSTRACTS, Band 86, no. 19, 9. Mai 1977, Columbus, Ohio, USA, K. ISHIMITSU et al. "Hydantoin fungicides", p. 163, Zusammenfassung Nr. 134 880w**
**HOUBEN-WEYL "Methoden der organischen Chemie", 4. Auflage, Band VIII: "Sauerstoffverbindungen III", 1952, GEORG THIEME VERLAG, Stuttgart, pp. 499, 680-684**
**HOUBEN-WEYL "Methoden der organischen Chemie", 4. Auflage, Band XI/1: "Stickstoffverbindungen II", 1957, GEORG THIEME VERLAG, Stuttgart, pp. 862-866**

(73) Patentinhaber: **Shell Agrar GmbH & Co. KG, Binger Strasse 173, D-6507 Ingelheim am Rhein (DE)**

(72) Erfinder: **Becher, Heinz-Manfred, Dr., Pfarrer-Heberer-Strasse 5, D-6530 Bingen/Rhein (DE)**
Erfinder: **Drandarevski, Christo, Dr., Boehringerstrasse 8, D-6507 Ingelheim/Rhein (DE)**
Erfinder: **Mengel, Rudolf, Dr., Im Herzenacker 32, D-6535 Gau Algesheim (DE)**
Erfinder: **Ost, Walter, Dr., Pfarrer-Heberer-Strasse 7, D-6535 Bingen/Rhein (DE)**

(74) Vertreter: **Hunter, Keith Roger Ian et al, 4 York Road, London SE1 7NA (GB)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Benzoylharnstoffe der allgemeinen Formel (I), Verfahren zu deren Herstellung sowie die Verwendung von Verbindungen der Formel (I) als insektizide Mittel.

Es ist bekannt, dass die Gruppe der Benzoylharnstoffe insektizid wirksam ist. (DE-AS 2 123 236). Diese bekannten Verbindungen, wie etwa N-(3,4,5-Trichlorphenyl)-N′-2,6-dichlorbenzoylharnstoff, befriedigen nicht immer im Hinblick auf Wirkungsstärke, Wirkungsprofil oder Toxizität.

Es wurde gefunden, dass Verbindungen der Formel (I) den aus dem Stand der Technik bekannten Benzoylharnstoffen in ihrer Wirkung überlegen sind.

Die Erfindung betrifft somit Verbindungen der Formel

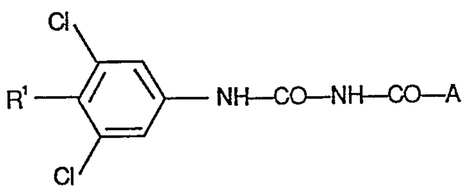
(I)

in der
$R^1$ für Fluor, Chlor, Brom oder Jod
A für 2,6-Difluorphenyl, 2-Chlorphenyl, 2-Chlor-6-fluorphenyl, 2,6-Dichlorphenyl oder 2-Fluorphenyl steht
mit der Einschränkung, dass die Kombination
$R^1$ für Chlor und
A für 2,6-Dichlorphenyl nicht mit umfasst ist.

Namentlich zu nennen sind somit als Vertreter der erfindungsgemässen Verbindungen (I):

N-(3,5-Dichlor-4-fluorphenyl)-N′-(2,6-difluorbenzoyl)-harnstoff
N-(3,4,5-Trichlorphenyl)-N′-(2,6-difluorbenzoyl)-harnstoff
N-(4-Brom-3,5-dichlorphenyl)-N′-(2,6-difluorbenzoyl)-harnstoff
N-(3,5-Dichlor-4-jodphenyl)-N′-(2,6-difluorbenzoyl)-harnstoff
N-(3,5-Dichlor-4-fluorphenyl)-N′-(2-Chlorbenzoyl)-harnstoff
N-(3,4,5-Trichlorphenyl)-N′-(2-Chlorbenzoyl)-harnstoff
N-(4-Brom-3,5-dichlorphenyl)-N′-(2-Chlorbenzoyl)-harnstoff
N-(3,5-Dichlor-4-jodphenyl)-N′-(2-chlorbenzoyl)-harnstoff
N-(3,5-Dichlor-4-fluorphenyl)-N′-(2,6-dichlorbenzoyl)-harnstoff
N-(4-Brom-3,5-dichlorphenyl)-N′-(2,6-dichlorbenzoyl)-harnstoff
N-(3,5-Dichlor-4-jodphenyl)-N′-(2,6-dichlorbenzoyl)-harnstoff
N-(3,5-Dichlor-4-fluorphenyl)-N′-(2-chlor-6-fluorbenzoyl)-harnstoff
N-(3,4,5-Trichlorphenyl)-N′-(2-chlor-6-fluorbenzoyl)-harnstoff
N-(4-Brom-3,5-dichlorphenyl)-N′-(2-chlor-6-fluorbenzoyl)-harnstoff
N-(3,5-Dichlor-4-jodphenyl)-N′-(2-chlor-6-fluorbenzoyl)-harnstoff
N-((3,5-Dichlor-4-fluorphenyl)-N′-(2-fluorbenzoyl)-harnstoff
N-(3,4,5-Trichlorphenyl)-N′-(2-fluorbenzoyl)-harnstoff
N-(4-Brom-3,5-dichlorphenyl)-N′-(2-fluorbenzoyl)-harnstoff und
N-(3,5-Dichlor-4-jodphenyl)-N′-(2-fluorbenzoyl)-harnstoff.

Die Herstellung der neuen Verbindungen kann nach an sich bekannten Verfahren erfolgen:

a) in dem man ein Anilin der Formel (II)

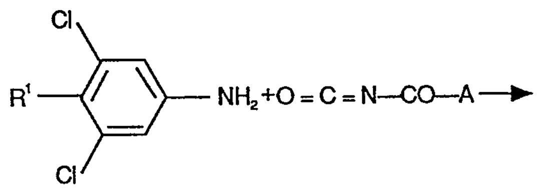
(I)

(II) (III)

mit einem Bezoylisocyanat der Formel (III) oder

b) ein Phenylisocyanat der Formel (IV) mit einem Benzamid der Formel (V)

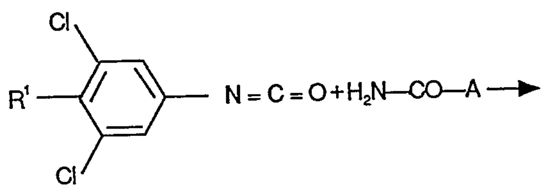
(I)

(IV) (V)

umsetzt.

Die Ausgangsverbindungen der Formel (II), (III), (IV) und (V) sind teilweise bekannt.

Verbindungen der Formel (II), in denen $R^1$ für Brom oder Jod steht, sind neu, wie auch Verbindungen der Formel (IV), in denen $R^1$ für Brom oder Jod steht, nämlich auch 4-Brom-3,5-dichlor anilin, 4-Jod-3,5-dichloranilin und 4-Brom-3,5-dichlorphenylisocyanat und 4-Jod-3,5-dichlorphenylisocyanat.

Die Isocyanate der Formel (IV) können hergestellt werden

a) durch Umsetzung der Aniline (II) mit Phosgen oder funktionellen Derivaten des Phosgens nach an sich bekannten Methoden oder

b) durch Curtius-Abbau ausgehend von einem reaktionsfähigen Säurehalogenid der Formel (VI), in der Hal für Fluor, Chlor, Brom oder Jod, vorzugsweise für Chlor oder Fluor steht. Das Säurehalogenid (VI) wird in einem inerten Lösungsmittel vorzugsweise Chlorbenzol, Toluol oder Xylol mit einem Azid, vorzugsweise Natrium- oder Kaliumazid, zu dem Säureazid der Formel (VII) umgesetzt. Wird die Reaktion bei Temperaturen oberhalb von etwa 100°C durchgeführt, isoliert man direkt das Isocyanat (IV).

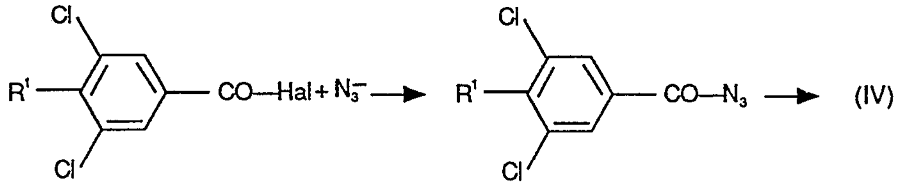


(VI) (VII)

Vorzugsweise wird die Reaktion so durchgeführt, dass zu einer auf Temperaturen über 100°C erwärmten Lösung des Säurehalogenids das Azid portionsweise zugegeben wird wobei das in situ erzeugte Azid VII sofort unter Stickstoffabspaltung zum Isocyanat der Formel (IV) weiterreagiert.

Azide der Formel (VII) können auch nach anderen literaturbekannten Verfahren hergestellt und anschliessend dem Abbau zum Isocyanat (IV) unterworfen werden, wie etwa durch Umsetzung von 4-Halogen-3,5-dichlor-benzoylhydrazid mt Nitrit zum Azid (VII).

Das Azid (VII) kann aber auch zunächst bei niedrigerer Temperatur hergestellt und dann gezielt bei höherer Temperatur zum Isocyanat (IV) umgelagert werden. Die Erfindung betrifft daher auch die neuen Carbonsäureazide der Formel (VII):

3,5-Dichlor-4-fluorbenzoesäureazid
3,4,5-Trichlorbenzoesäureazid
4-Brom-3,5-dichlorbenzoesäureazid und
3,5-Dichlor-4-jodbenzoesäureazid.

Hervorzuheben ist, dass das 3,5-Dichlor-4-fluor-benzoesäureazid (VII, $R^1$ = F) besonders vorteilhaft aus dem gut verfügbaren 3,4,5-Trichlorbenzoylchlorid (VI, $R^1$ = Cl, Hal = Cl) durch doppelten Halogenaustausch herstellbar ist.

Bei der Umsetzung von 3,4,5-Trichlorbenzoylchlorid mit Fluoriden, wie KF, kann in einem Reaktionsschritt sowohl das Chloratom in 4-Position des aromatischen Systems wie auch das Chloratom aus der Carbonsäurefunktion ausgetauscht werden. Das so darstellbare 3,5-Dichlor-4-fluorbenzoylfluorid (VI) reagiert unter besonders schonenden Bedingungen mit guter Ausbeute zum entsprechenden Azid (VII).

Die Aniline der Formel (II) können in zweistufiger Reaktionsfolge ausgehend vom 2,6-Dichloranilin (VIII) hergestellt werden, wobei in der ersten Reaktionsstufe die Aminogruppe nach an sich bekannten Verfahren durch die Halogene Fluor, Chlor, Brom oder Jod substituiert wird und das so erhaltene 4-Halogen-3,5-dichlornitrobenzol (IX) in der

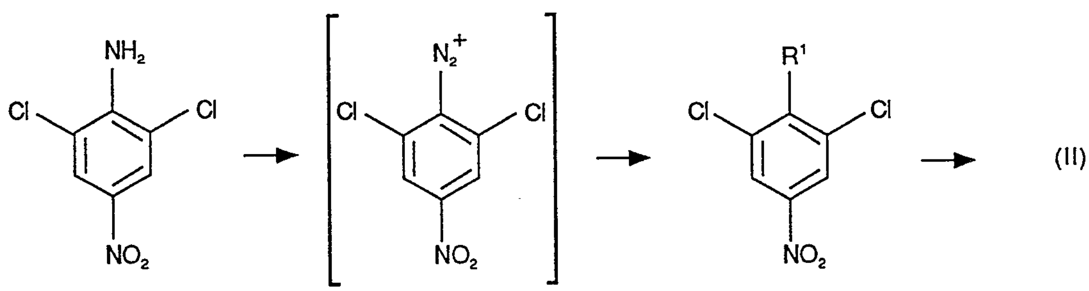


(VIII) (IX)

zweiten Stufe zum Anilin (II) reduziert wird.

Benzoylisocyanate der Formel III können aus den entsprechenden Benzamiden und Oxalylchlorid erhalten werden.

Die Benzoylharnstoffe der Formel I sind geeignet, Schädlinge aus der Klasse der Insekten und Spinnentiere wirksam zu bekämpfen. Sie können im Pflanzenschutz sowie auf dem Hygiene-, Vorratsschutz- und Veterinärsektor als Schädlingsbekämpfungsmittel eingesetzt werden. Sie wirken vorzugsweise auf die Entwicklungsstadien ein, bei denen eine Häutung stattfindet (sog. Chitinsynthese-Hemmung).

Zu den schädlichen Insekten gehören aus der Ordnung der Lepidoptera beispielsweise Plutella maculipennis, Leucoptera coffealla, Hyponomeuta malinellus, argyresthia conjugella, Sitotroga cerealella, Phthorimaea operculella, Capua reticulana, Sparganothis pilleriana, Cacoecia murinana, Tortrix viridana, Clysia ambiguella, Evetria buoliana, Polychrosis botrana, Cydia pomonella, Laspeyresia molesta, Laspeyresia funebrana, Ostrinia nubilalis, Loxostege sticticalis, Ephestia kuehniella, Chilo suppressalis, Galleria Mellonella, Malacosoma neustria, Dendrolimus pini, Thaumatopoea pityocampa, Phalera bucephala, Cheimatobia brumata, Hibernis defoliaria, Bupalus piniarus, Hyphantria cunea, Agrotis segetum, Agrotis ypsilon, Barathra brassicae, Cirphis unipuncta, Prodenia litura, Laphygma exigua, Panolis flammea, Earias insulana, Plusia gamma, Alabama argillacea, Lymantria dispar, Lymantria monacha, Pieris brassicae, Aporia crataegi, Pectinophora gossypielle, Heliothis virescens; aus

der Ordnung der Coleoptera beispielsweise Epilachna varivestris, Phyllopertha horticola, Crioceris asparagi, Lemma melanopus, Leptinotarsa decemlineata, Diabrotica 12-punctata, Anthonomus grandis, Sitophilus granaria, aus der Ordnung der Diptera beispielsweise Rhagoletis cerasi, Rhagoletis pomonella, Anopheles maculipennis, Culex pipiens, Aedes aegypti, Aedes vexans, Musca domestica, Fannia canicularis, Muscina stabulans, Glossina morsitans, Lucilia cuprina, Lucilia sericata, Hypoderma lineata; aus der Ordnung der Hymenoptera beispielsweise Cephalcia abietis, Diption pini, Diprion sertifer, Pristiphora abientina; Schädlinge aus der Ordnung der Heteroptera; aus der Ordnung der Homoptera beispielsweise Bemisia tabaci, Psylla piri, Trialeurodes vaporariorum.

Zur Klasse der Arachnoidea gehören Spinnentiere (Acarina), beispielsweise Phyllocoptranta oleivora.

Ausserdem können die Verbindungen der Formel I auch als Schneckenbekämpfungsmittel zur Bekämpfung, landlebender Nackt- und Gehäuseschnecken verwendet werden.

Zu nennen sind als Vertreter der Nacktschnekken Arion rufus, Arion ater und andere Arionidae, Limax-Arten und die Ackerschnecken wie Deroceras reticulatum und D. agreste aus der Familie Milacidae.

Weiterhin werden auch die Gehäuseschnecken u. a. der Gattungen Bradybaena, Cepaea, Cochlodina, Discus, Euomphalia, Galba, Helicigona, Helix, Helicella, Helicodiscus, Lymnaea, Opeas, Vallonia und Zonitoides geschädigt.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln Streumitteln, Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphtaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wässrige Anwendungsformen können aus Emulsionskonzentraten, Suspensionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier-, oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Für die Anwendung werden die erfindungsgemässen Wirkstoffe mit den üblichen Hilfs- und/oder Trägerstoffen zu gebräuchlichen Formulierungen verarbeitet, z.B. Emulsionskonzentraten, Suspensionspulvern, Stäuben, Suspensionskonzentrate.

Die Anwendung erfolgt in Spritzbrühen und Stäuben mit Wirkstoffkonzentrationen zwischen etwa 0,0005 und 2%, in Form von ULV-Formulierungen auch mit höheren Wirkstoffkonzentrationen (bis etwa 90%). Die Aufwandmenge pro Hektar beträgt je nach Wirkstoff und Kultur zwischen etwa 0,005 und 0,5 kg, vorzugsweise zwischen 0,01 und 0,25 kg.

Formulierungsbeispiel:

Suspensionspulver (Angaben in Gew.-%)
25% Wirkstoff gemäss der Erfindung
55% Kaolin
10% kolloidale Kieselsäure
9% Ligninsulfonat (Dispergiermittel)
1% Natriumtetrapropylenbenzolsulfonat (Netzmittel)

Die Bestandteile werden wie üblich zu einem Suspensionspulver (Partikelgrösse: 4 µm) verarbeitet. Für die Anwendung wird mit Wasser eine Spritzbrühe hergestellt, die etwa 0,0005 bis 0,5 Gew.-% Wirkstoff enthält.

Herstellung der Ausgangsverbindungen

Beispiel 1

3,4,5-Trichlorphenylisocyanat (IV) aus 3,4,5-Trichloranilin (II)

In eine Lösung von 3,4,5-Trichloranilin (II) (29,6 g) in Toluol (300 ml) wird unter Rühren bei Raumtemperatur Chlorwasserstoffgas eingeleitet. In die so erhaltene Aufschlämmung von 3,4,5-Trichloranilin-Hydrochlorid (II) wird Phosgen (30 g) eingeleitet und anschliessend so lange zum Sieden erwärmt, bis sich das Reaktionsgemisch geklärt hat. Um ein Entweichen des Phosgens zu vermeiden, muss ein Intensivrückflusskühler verwendet werden.

Nach nahezu vollständiger Klärung des Reaktionsgemisches wird von den geringen Rückständen abgesaugt, das Filtrat wird eingeengt und das ölige Isocyanat isoliert.

Ausbeute: 32,1 g der Titelverbindung als Öl.

Beispiel 2

3,4,5-Trichlorphenylisocyanat (IV) aus 3,4,5-Trichlorbenzoylchlorid (VI)

Zu einer auf 125°C erwärmten Suspension von Natriumazid (7,0 g) in Chlorbenzol (300 ml) wird unter Rühren eine Lösung von 3,4,5-Trichlorbenzoylchlorid (24,4 g) (VI) in Chlorbenzol (100 ml) zugetropft, wobei Stickstoff frei wird.

Nach beendeter Zugabe und Abklingen der Stickstoff-Entwicklung wird noch 2 Stunden bei 125°C gerührt, mit Kieselgel (3,0 g) versetzt und nach Erkalten auf Raumtemperatur filtriert.

Nach Abdestillieren des Lösungsmittels fällt die Titelverbindung als Öl an.

Ausbeute: 22,1 g der Titelverbindung als Öl

Anstelle von Chlorbenzol kann auch Toluol oder Xylol als Lösungsmittel verwendet werden, wobei gewünschtenfalls zur Temperaturerhöhung auch unter Überdruck gearbeitet werden kann.

Beispiel 3

4-Halogen-3,5-dichloranilin (II) aus 2,6-Dichlor-4-nitroanilin (III) über 4-Halogen-3,5-dichlornitrobenzol (IX)

Beispiel 3a

3,4,5-Trichlornitrobenzol (Stufe 1) (IX)

Zu einer auf −5°C gekühlten Lösung von 2,6-Dichlor-4-nitroanilin (VIII) (124,2 g) in konzentrierter Phosphorsäure (250 ml), Eisessig (75 ml) und konz. Schwefelsäure (500 ml) wird in kleinen Portionen Nitrosylhydrogensulfat (84,0 g) zugegeben. Dann wird noch 1 Stunde bei 0°C, 1 Stunde bei 5-10°C und 1 Stunde bei 15°C nachgerührt. In die so erhaltene Lösung wird unter intensivem Rühren eine auf 45°C erwärmte Kupfer-(I)-chloridlösung (0,80 Mol) zugetropft. Dann wird noch 1 Stunde bei 50-60°C nachgerührt und das Reaktionsgemisch in Eis (2 kg) eingegossen. Nach Extraktion mit Dichlormethan isoliert man 3,4,5-Trichlornitrobenzol (IX).

Ausbeute: 101 g (74%) 3,4,5-Trichlornitrobenzol (IX) vom Schmp. 68–70°C

Eine Lösung von 3,4,5-Trichlornitrobenzol (34 g) (IX) in Essigester (350 ml) wird nach Zusatz von 4,0 g Raney-Nickel unter Normaldruck bei Raumtemperatur hydriert. Nach beendeter Wasserstoffaufnahme wird vom Katalysator abgesaugt und das Filtrat eingeengt.

Ausbeute: 25,7 g (87%) 3,4,5-Trichloranilin (II) vom Schmp. 97–98°C.

Beispiel 3b

4-Brom-3,5-dichlornitrobenzol (IX) (Stufe 1)

Analog Beispiel 3a unter Verwendung von Kupfer (I)-bromid-Lösung. Ausbeute: 59% d. Th. 4-Brom-3,5-dichlornitrobenzol (IX) vom Schmp. 88–90°C.

4-Brom-3,5-dichloraniline (II) (Stufe 2)

Zu einer auf Siedetemperatur erwärmten Lösung von 4-Brom-3,5-dichlornitrobenzol (IX) (27,1 g) in Aceton (700 ml) wird unter Rühren eine Lösung von Zinn-(II)-chlorid-dihydrat (75,0 g) in konz. Salzsäure (150 ml) getropft und noch 30 min. zum Sieden erhitzt. Nach Abdestillieren des Lösungsmittels wird auf Eis gegossen, durch Zugabe von Natronlauge (10%ig) alkalisch gestellt und mit Ether extrahiert. Die vereinigten Etherextrakte werden eingeengt und an Kieselgel mit Methylenchlorid gereinigt.

Ausbeute: 14,7 g (61%) 4-Brom-3,5-dichloranilin (II) vom Schmp. 121–123°C.

Beispiel 3c

4-Jod-3,5-dichlornitrobenzol (IX) (Stufe 1)

Zu einer auf 5°C gekühlten Lösung von 2,6-Dichlor-4-nitroanilin (26,5 g) (VIII) in konz. Schwefelsäure (200 ml) wird unter Rühren Natriumnitrit (12,0) in kleinen Portionen eingetragen. Danach wird noch 1 Stunde bei 8–10°C weitergerührt und dann auf Eis (800 g) gegossen und mit einer wässrigen Kaliumiodidlösung (30 g KJ in 60 ml Wasser) versetzt, wobei die Mischung stark schäumt. Es wird noch 1 Stunde bei Raumtemperatur nachgerührt, mit Ether extrahiert und die vereinigten Etherextrakte werden nach Einengen an Kieselgel mit Dichlormethan gereinigt.

Ausbeute: 25,0 g (58%) 4-Jod-3,5-dichlornitrobenzol vom Schmp. 153–158°C.

4-Jod-3,5-dichloranilin (II) (Stufe 2)

Ausgehend von 4-Jod-3,5-dichlornitrobenzol (IX) wird analog Beispiel 3b 4-Jod-3,5-dichloranilin (II) vom Schmp. 147–148°C in 85% Ausbeute dargestellt.

Beispiel 4

Synthese von Benzoylharnstoffen der Formel (I) ausgehend von 4-Halogen-3,5-dichloranilin und Benzoylisocyanaten der Formel (III)

Beispiel 4a

N-(3,4,5-Trichlorphenyl)-N′-(2,6-difluorbenzoyl)-harnstoff (I)

3,4,5-Trichloranilin (19,7 g) (II) und 2,6-Difluorbenzoylisocyanat (18,3 g) (III) werden bei 50°C in Toluol (200 ml) gelöst und 5 Stunden bei dieser Temperatur belassen.

Nach Erkalten auf Raumtemperatur wird die Titelverbindung abgesaugt und getrocknet.

Ausbeute: 34,2 g (90%) der Titelverbindung als Kristalle vom Schm. 231-232 °C

Analog darstellbar sind die in nachstehender Tabelle zusammengefassten Verbindungen der Formel I

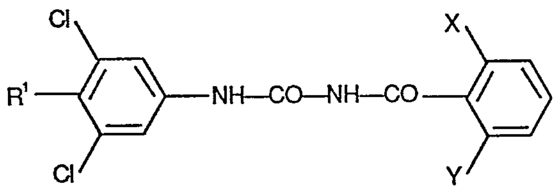

| No. | $R^1$ | X | Y | Schmp.[°C] |
|---|---|---|---|---|
| 4b | F | F | F | 209-212° |
| 4c | Cl | F | Cl | 248-251° |
| 4d | Cl | H | Cl | 253-255° |
| 4e | Br | F | F | 230-233° |
| 4f | Br | F | Cl | 240-242° |
| 4g | Br | H | Cl | 243-245° |
| 4h | J | F | F | 243-245° |
| 4i | J | F | Cl | 245-247° |
| 4j | J | H | Cl | 257-259° |
| 4k | F | Cl | Cl | |
| 4l | Br | Cl | Cl | |
| 4m | J | Cl | Cl | |
| 4n | F | H | Cl | |
| 4o | F | Cl | F | |
| 4p | F | H | F | |
| 4q | Cl | H | F | |
| 4r | Br | H | F | |
| 4s | J | H | F | |

Beispiel 5

Synthese von Benzoylharnstoffen der Formel (I) ausgehend von 4-Halogen-3,5-dichlorphenylisocyanaten (IV) und Benzamiden der Formel (V).

Beispiel 5a

N-(3,4,5-Trichlorphenyl)-N′-(2,6-difluorbenzoyl)harnstoff (I)

Eine Lösung von 3,4,5-Trichlorphenylisocyanat (22,3 g) (IV), 2,6-Difluorbenzamid (15,7 g) (V) in Toluol (200 ml) wird 8 Stunden auf 90-95 °C erwärmt. Aus der zunächst klaren Lösung kristallisiert die Titelverbindung langsam aus und wird nach Erhalten des Reaktionsgemisches abgesaugt.

Ausbeute: 34,5 g (90%) der Titelverbindung vom Schmp. 231-232 °C.

Analog darstellbar sind die in nachstehender Tabelle zusammengefassten Verbindungen der Formel I

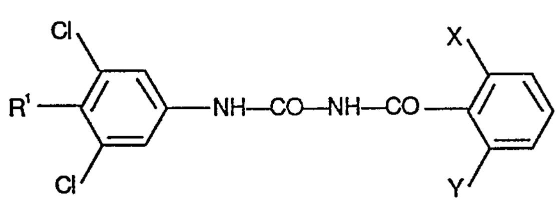

| No. | $R^1$ | X | Y | Schmp.[°C] |
|---|---|---|---|---|
| 5b | F | F | F | 209-212° |
| 5c | Cl | F | Cl | 248-251° |
| 5d | Cl | H | Cl | 253-255° |
| 5e | Br | F | F | 230-233° |
| 5f | Br | F | Cl | 240-242° |
| 5g | Br | H | Cl | 243-245° |
| 5h | J | F | F | 243-245° |
| 5i | J | F | Cl | 245-247° |
| 5j | J | H | Cl | 257-259° |
| 5k | F | Cl | Cl | |
| 5l | Br | Cl | Cl | |
| 5m | J | Cl | Cl | |
| 5n | F | H | Cl | |
| 5o | F | Cl | F | |
| 5p | F | H | F | |
| 5q | Cl | H | F | |
| 5r | Br | H | F | |
| 5s | J | H | F | |

## Patentansprüche

1. Verbindungen der Formel

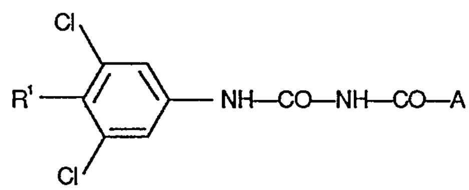 (I)

in der

$R^1$ für Fluor, Chlor, Brom oder Jod

A für 2,6-Difluorphenyl, 2-Chlorphenyl, 2-Chlor-6-fluorphenyl, 2,6-Dichlorphenyl, 2-Fluorphenyl steht,

mit der Einschränkung, dass die Kombination

$R^1$ für Chlor und

A für 2,6-Dichlorphenyl nicht mit umfasst ist.

2. Insektizides Mittel, gekennzeichnet durch einen Gehalt an einer Verbindung der Formel I.

3. Verfahren zur Bekämpfung von Insekten, dadurch gekennzeichnet, dass man Verbindungen der Formel (I) auf den Lebensraum der Insekten einwirken lässt.

4. Verfahren zur Herstellung von Verbindungen nach Anspruch 1,

a) durch Umsetzung eines Anilins der Formel (II) mit einem Bezoylisocyanat der Formel (III)

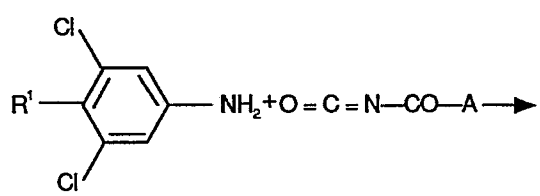 (I)

(II) (III)

oder

b) durch Umsetzung eines Phenylisocyanats der Formel (IV) mit einem Benzamid der Formel (V)

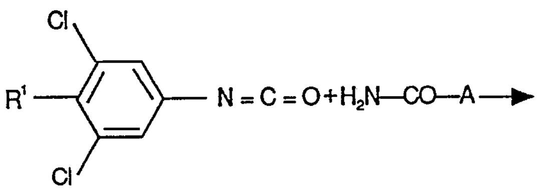

(I)

(IV) (V)

wobei A und $R^1$ wie zuvor definiert sind.

5. Carbonsäureazide der Formel

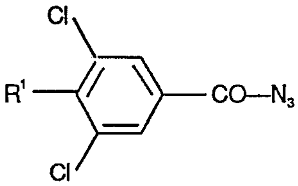

(VII)

in der $R^1$ für Fluor, Chlor, Brom oder Jod steht.

6. Verfahren zur Herstellung von Isocyanaten der Formel (IV), in der $R^1$ für Fluor, Chlor, Brom oder Jod steht, durch thermische Umlagerung des in situ erzeugten Azids der Formel (VII)

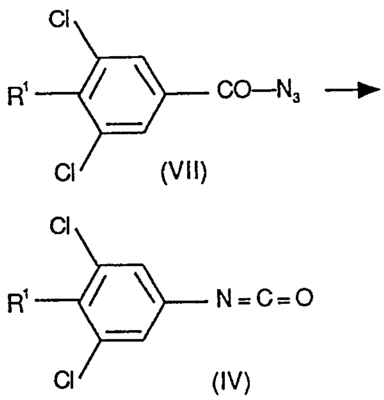

(VII)

(IV)

bei Temperaturen über 100 °C.

7. Schneckenbekämpfungsmittel gekennzeichnet, durch einen Gehalt an einer Verbindung der Formel I.

8. Verfahren zur Bekämpfung landlebender Schnecken, dadurch gekennzeichnet, dass man Verbindungen der Formel (I) auf den Lebensraum der Schnecken einwirken lässt.

**Revendications**

1. Composés de formule

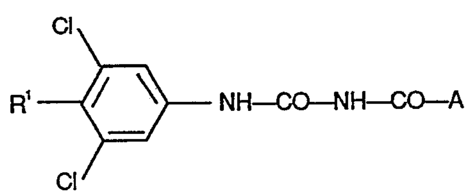

(I)

dans laquelle

$R^1$ représente le fluor, le chlore, le brome ou l'iode,

A représente un groupe 2,6-difluorophényle, 2-chlorophényle, 2-chloro-6-fluorophényle, 2,6-dichlorophényle, 2-fluorophényle,

étant spécifié que la combinaison suivante:

$R^1$ représente le chlore et

A représente le groupe 2,6-dichlorophényle, est exclue.

2. Produit insecticide caractérisé en ce qu'il contient un composé de formule I.

3. Procédé pour combattre les insectes, caractérisé en ce que l'on fait agir des composés de formule I sur l'habitat des insectes.

4. Procédé de préparation des composés selon la revendication 1,

a) par réaction d'une aniline de formule II avec un isocyanate de benzoyle de formule III

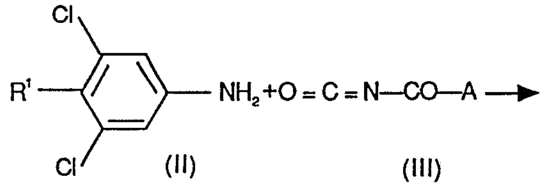

(I)

(II) (III)

ou bien

b) par réaction d'un isocyanate de phényle de formule IV avec un benzamide de formule V

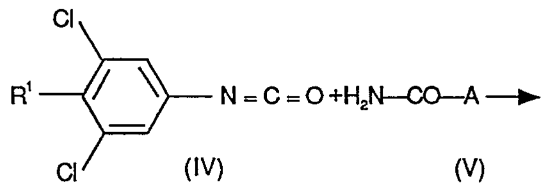

(I)

(IV) (V)

A et $R^1$ ayant les significations indiquées ci-dessus.

5. Azides d'acides carboxyliques de formule

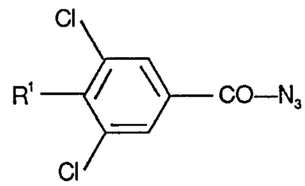

(VII)

dans laquelle $R^1$ représente le fluor, le chlore, le brome ou l'iode.

6. Procédé de préparation des isocyanates de formule IV dans laquelle $R^1$ représente le fluor, le chlore, le brome ou l'iode, par transposition à la chaleur de l'azide – formé in situ – de formule VII

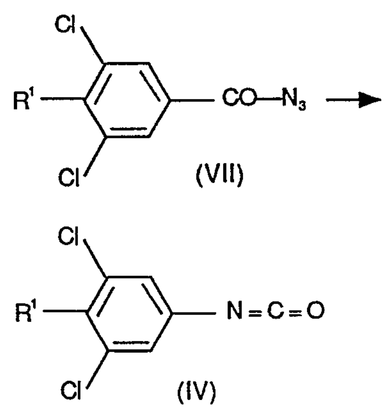

(VII)

(IV)

à des températures supérieures à 100 °C.

7. Produit pour combattre les gastéropodes, caractérisé en ce qu'il contient un composé de formule I.

8. Procédé pour combattre les gastéropodes terrestres, caractérisé en ce que l'on fait agir des composés de formule I sur l'habitat des gastéropodes.

## Claims

1. Compounds of the formula

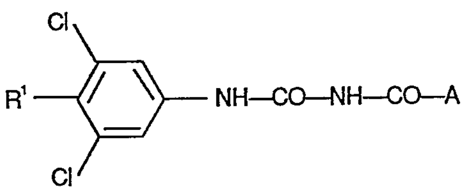

(I)

wherein

$R^1$ represents fluorine, chlorine, bromine or iodine

A represents 2,6-difluorophenyl, 2-chlorophenyl, 2-chloro-6-fluorophenyl, 2,6-dichlorphenyl, 2-fluorophenyl,

with the limitation that the combination

$R^1$ representing chlorine and

A representing 2,6-dichlorphenyl is not included.

2. Insecticidal agent, characterized by a content of a formula (I) compound.

3. Process for controlling insects characterized in that formula (I) compounds are made to act on the insects' habitat.

4. Process for producing compounds as claimed in claim 1,

a) by reacting a formula (II) aniline with a formula (III) benzoyl isocyanate

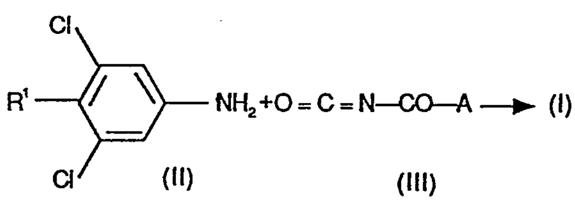

(II) (III) (I)

b) by reacting a formula (IV) isocyanate with a formula (V) benzamide

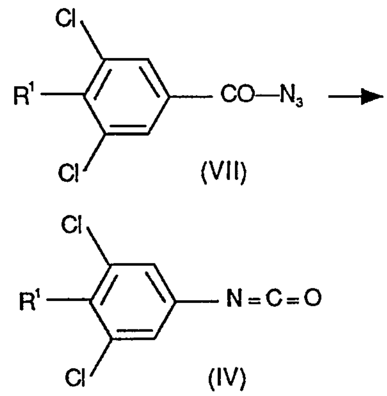

(VII) (IV)

with A and $R^1$ being defined as hereinabove.

5. Carboxylic acid azides of the formula

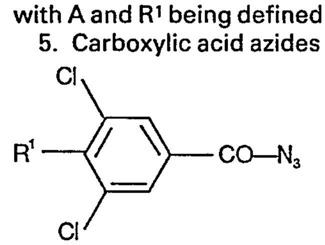

(VII)

wherein $R^1$ represents fluorine, chlorine, bromine or iodine.

6. Process for producing isocyanates of formula (IV) wherein $R^1$ represents fluorine, chlorine, bromine or iodine by thermal transposition of the in-situ generated formula (VII) azide.

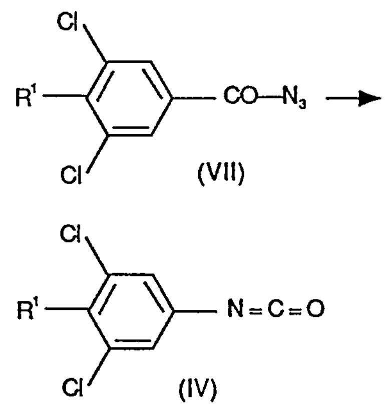

(VII) (IV)

at temperatures higher than 100°C.

7. Molluscicide characterized by a content of a formula I compound.

8. Process for the control of land snails, characterized in that formula (I) compounds are made to act on the snails' habitat.